Europäisches Patentamt

European Patent Office

Office européen des brevets

(11) Publication number: **0 081 836**
**B1**

# (12) EUROPEAN PATENT SPECIFICATION

(45) Date of publication of patent specification: **05.03.86**

(51) Int. Cl.⁴: **A 61 K 31/47**

(21) Application number: **82111539.1**

(22) Date of filing: **13.12.82**

(54) Use of the compound 9-(p-(N-methylacetamido)anilino)-7-methyl-1H-imidazo(4,5-f)quinoline hydrochloride (I) as antitumor agent.

(30) Priority: **14.12.81 US 330577**

(43) Date of publication of application:
**22.06.83 Bulletin 83/25**

(45) Publication of the grant of the patent:
**05.03.86 Bulletin 86/10**

(84) Designated Contracting States:
**AT BE CH DE FR GB IT LI LU NL SE**

(56) References cited:
**US-A-3 878 206**

(73) Proprietor: **Norwich Eaton Pharmaceuticals, Inc.**
**17 Eaton Avenue**
**Norwich New York 13815 (US)**

(72) Inventor: **Ebetino, Frank F.**
**14 Summit Street**
**Norwich, New York 13815 (US)**

(74) Representative: **Wuesthoff, Franz, Dr.-Ing. et al**
**Patentanwälte Wuesthoff -v. Pechmann-**
**Behrens-Goetz Schweigerstrasse 2**
**D-8000 München 90 (DE)**

Courier Press, Leamington Spa, England.

## Description

This invention is concerned with the use of the compound 9-[p-(N-methylacetamido)-anilino]-7-methyl-1H-imidazo [4,5-f] quinoline hydrochloride (I) for preparing parenterally injectable solutions for treating malignant tumors in a host afflicted therewith. That compound is described in U.S. patent No. 3,878,206 wherein its utility as an anti-bacterial agent is disclosed.

It has now been found that (I) possesses noteworthy antitumor activity. Such activity is evinced in those systems currently regarded and acknowledged by those skilled in the art to unearth candidates for successful use as antitumor agents and to be reasonable predictors of such use in a variety of species.

Such systems comprise murine tumors such as P—388 lymphocytic leukemia, B—16 melanocarcinoma and L—1210 lymphoid leukemia (Cancer Clinical Trials, 1979; 2:195—216; Recent Results in Cancer Research, Vol. 70.)

It has been discovered that when (I) is administered parenterally as a solution in isotonic saline to a host harboring one of the aforecited tumors, a salutary effect is elicited. The results are depicted in the following table:

### Antitumor Activity[a]

| L—1210 | | | | P—388 | | | | B—16 | | | |
| Limphoid Leukemia[b] | | | | Lymphocytic Leukemia[b] | | | | Melanocarcinoma[b] | | | |
| Dose[c] | ILS[d] | T/C[e] | T-C[f] | Dose[c] | ILS[d] | T/C[e] | T-C[f] | Dose[c] | ILS[d] | T/C[e] | T-C[f] |
|---|---|---|---|---|---|---|---|---|---|---|---|
| 50 | 43 | 143 | −2.3 | 50 | 46 | 146 | −3.0 | 50 | 49 | 149 | −0.7 |
| 12.5 | 25 | 125 | 1.9 | 25 | 43 | 143 | −1.0 | 25 | 98 | 198 | −0.4 |
| | | | | 12.5 | 34 | 134 | −0.8 | 50 | 84 | 184 | −2.3 |
| | | | | 6.25 | 26 | 126 | −0.8 | | | | |

a. Protocols and tumor systems described in Geran, et al. Cancer Chemother. Rep. Part 3. 3 (No. 2) 1 (1972).
b. Intraperitoneal-tumor implantation, intraperitoneal QD1—9 treatment schedule.
c. In mg/kg/injection in isotonic saline.
d. Increased life span = T/C — 100 in percent.
e. T/C = ratio (expressed as %) of the mean survival time of the treated group divided by the mean survival time of the control group.
f. T-C = average weight change of test group minus average weight change of control animals in grams.

The values such as ILS and T/C depicted in the foregoing table demonstrate the efficiency of (I) in the various antitumor systems.

## Claim

The use of the compound 9-[p-(N-methylacetamido)anilino]-7-methyl-1H-imidazo[4,5-f]quinoline hydrochloride for preparing parenterally injectable solutions for treating malignant tumors in a host afflicted therewith.

## Revendication

Utilisation de l'hydrochlorure de la 9-(p-(N-méthlacétamido)anilino)7-méthyl-1H-imidazo(4,5-f)quinoléine pour la préparation de solutions á injections parenterales pour le traitement de tumeurs malignes dans un hôte en souffrant.

## Patentanspruch

Verwendung der Verbindung 9-[p-(N-Methylacetamido)anilino]7-methyl-1H-imidazo[4,5-f]chinolin-hydrochlorid zur Herstellung von parenteral injizierbaren Lösungen zur Behandlung bösartiger Tumoren bei einem davon befallenen Wirt.